(19) Europäisches Patentamt
European Patent Office
Office européen des brevets

(11) EP 2 181 763 B1

(12) **EUROPEAN PATENT SPECIFICATION**

(45) Date of publication and mention of the grant of the patent:
**20.06.2012 Bulletin 2012/25**

(21) Application number: **09174399.7**

(22) Date of filing: **28.10.2009**

(51) Int Cl.:
***B01J 31/04*** *(2006.01)* ***C07C 67/26*** *(2006.01)*

# (54) Catalyst and process for alkoxylation

Katalysator und Verfahren zur Alkoxylierung

Catalyseur et procédé d'alcoxylation

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **29.10.2008 EP 08167851**

(43) Date of publication of application:
**05.05.2010 Bulletin 2010/18**

(73) Proprietor: **Shell Internationale Research Maatschappij B.V.**
**2596 HR The Hague (NL)**

(72) Inventors:
- **Duijghuisen, Henricus Petrus Bernardus**
  **1031 CM Amsterdam (NL)**
- **Van der Heijden, Harry**
  **1031 CM Amsterdam (NL)**
- **Van der Made, Renate Helena**
  **1031 CM Amsterdam (NL)**
- **On, Quoc An**
  **1031 CM Amsterdam (NL)**
- **Van Zon, Arie**
  **1031 CM Amsterdam (NL)**

(74) Representative: **Matthezing, Robert Maarten**
**Shell International B.V.**
**Intellectual Property Services**
**P.O. Box 384**
**2501 CJ The Hague (NL)**

(56) References cited:
**EP-A- 0 252 250** **EP-A- 0 424 066**
**WO-A-02/38269** **US-A1- 2005 240 064**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 181 763 B1

## Description

Field of the Invention

The present invention relates to a catalyst and process for alkoxylation.

Background of the Invention

A large variety of products useful, for instance, as non-ionic surfactants, wetting and emulsifying agents, solvents and chemical intermediates are prepared by the addition reaction (alkoxylation reaction) of alkylene oxides (epoxides) with organic compounds having one or more active hydrogen atoms.

For example, alcohol ethoxylates may be prepared by the reaction of ethylene oxide with aliphatic alcohols of 6 to 30 carbon atoms. Such ethoxylates, and to a lesser extent the corresponding propoxylates and compounds containing mixed oxyethylene and oxypropylene groups, are widely employed as non-ionic detergent components in cleaning and personal care formulations.

One typical method of preparing such alkoxylated alcohols is by hydroformylating an olefin into an oxo-alcohol, followed by alkoxylation of the resulting alcohol by reaction with a suitable alkylene oxide such as ethylene oxide or propylene oxide.

Alkylene oxide addition reactions are known to produce a product mixture of various alkoxylate groups having different numbers of alkylene oxide adducts (oxyalkylene adducts). The adduct number is a factor which in many respects controls the properties of the alkoxylate molecule, and efforts are made to tailor the average adduct number of a product and/or the distribution of adduct numbers within a product to the product's intended service.

Alkoxylate mixtures in which a relatively large proportion of the alkoxylate molecules have a number (n) of alkylene oxide adducts that is within a relatively narrow range of values have been reported in the art as being preferred for use in certain detergent formulations (GB-A-1462134; Research Disclosure No. 194010). Narrow-range alkoxylates are also known to be particularly valuable as chemical intermediates in the synthesis of certain carboxyalkylated alkyl polyethers (US-A-4098818) and of certain alkyl ether sulfates (GB-A-1553561).

US-A-5840995 discloses a narrow-range alkoxylation catalyst comprising a mixture of an active hydrogen containing compound and a reaction product of the components comprising:

(a) a compound of the formula $C_8$-$C_{22}$ -alkyl-O(AO)$_x$-B wherein A is -$C_2H_4$- or -$C_3H_6$-, B is hydrogen or a group of the formula -$CH_2COOH$, -$SO_3H$, or -$PO(OH)_2$ and x is from 2 to 10;
(b) an alkaline earth metal compound; and
(c) sulphuric acid; and

a process for alkoxylating an active hydrogen-containing compound using said catalyst.

There is increasing interest in alternative routes to non-ionic surfactants from renewable feedstocks such as alkoxylates of fatty acid esters, in particular to narrow-range alkoxylates of fatty acid esters.

The direct alkoxylation of fatty acid alkyl esters in the presence of a catalyst occurs by selectively inserting an alkylene oxide molecule between the carbonyl carbon atom and the ester-bonded alkoxy group.

EP-A-0335295 discloses a method for the preparation of carboxylic acid esters, in particular fatty acid esters, from alkylene oxides, wherein the carboxylic acid ester is of formula, $RC(O)OR^1$, where R an alkyl radical with 1 to 25 carbon atoms or an alkenyl radical with 2 to 25 C-atoms and $R^1$ is an alkyl radical with 1 to 10 carbon atoms, with an alkylene oxide selected from the group of ethylene oxide, propylene oxide and butylene oxide in presence of a catalyst at a temperature from 100 to 200 °C with direct incorporation of the alkylene oxide in the carboxylic acid ester, characterised in that the conversion is conducted with a catalyst selected from an alkali metal or an alkaline earth metal compound from the group of the hydroxides, oxides and alcoholates.

WO-A-02/38269 describes a narrow-range alkoxylation catalyst having high activity, being in the form of a visually homogeneous, liquid suspension or a homogeneous paste, which catalyst contains:

(a) calcium salts of low molecular carboxylic and/or hydroxycarboxylic acids and/or hydrates of the former,
(b) a strong oxy-acid, preferably sulphuric acid,
(c) an alcohol and/or an ester,

or products of their reciprocal reactions, being formed on homogenisation, a total concentration of the calcium salts and sulphuric acid being from 10 % to 65 % by weight (wt. %).

Said alkoxylation catalyst is said to show high activity which, at low concentrations, enables very good dispersion in the alkoxylation reaction medium, to eventually minimise the duration of the reaction initiation and to obtain a high

conversion rate of alkylene oxide. It is further indicated in WO-A-02/38269 that the possibility to use the catalyst at very low concentrations has a favourable effect on the quality of the resulting products and helps reduce the possible costs of removal of the catalyst residue.

WO-A-2005/115964 discloses a method to minimise the initial induction period of a batch alkoxylation process, in particular during the catalytic oxyethylation of esters (reactants) in a periodic reactor, said method comprising introduction of ethylene oxide into the reactor filled with the reactant and in the presence of a catalyst, characterized in that the reactor is filled with a portion (x') of a pre-designed total quantity (X) of the reactant to be subjected to oxyethylation and with a total pre-designed quantity (Y) of the catalyst, whereupon another portion (z') of the pre-designed total quantity (Z) of ethylene oxide is fed to the reactor to activate the catalyst and induce a reaction and after the introduced quantity (z') of ethylene oxide has been converted entirely or partly, the reactant feed is supplemented to the pre-designed quantity (X) and ethylene oxide is continued to be fed until the pre-designed quantity (Z) has been introduced, preferably, the portion (x') of the reactant introduced in the initial phase of the synthesis is as small as possible relative to the total pre-designed reactant feed (X), that is, its quantity is around the minimum feed specified for a given reactor, that is, when the value of the quotient x'/X is as small as possible.

However, whilst fatty acid methyl esters (FAME) can be efficiently alkoxylated using Ca-catalysts and reaction conditions according to WO-A-02/038269 and WO-A-2005/115964, as outlined hereinabove, it has been found that the resulting ester alkoxylates, in particular methyl ester ethoxylates (MEE) display distinct discolouration and in particular, yellowing.

It is therefore highly desirable to be able to produce narrow range ester alkoxylates from renewable sources, wherein said alkoxylates do not have adverse colouration. In particular, this would considerably increase flexibility in the detergents industry, which generally aims to produce water-white (colourless) liquid detergent formulations or white detergent powder formulations.

## Summary of the Invention

Accordingly, in one embodiment of the present invention there is provided a catalyst composition for alkoxylation, said catalyst composition comprising,

(a) one or more alkaline earth metal salts of carboxylic and/or hydroxycarboxylic acids and/or hydrates of the former,
(b) an oxy-acid selected from sulphuric acid and ortho-phosphoric acid,
(c) an alcohol and/or an ester,
(d) a peroxy acid and/or a salt thereof

and/or products of the reciprocal reactions of (a), (b), (c) and/or (d).

In another embodiment of the present invention, there is provided a method of preparing said catalyst composition, wherein (a) one or more alkaline earth metal salts of carboxylic and/or hydroxycarboxylic acids and/or hydrates of the former, (b) an oxy-acid selected from sulphuric acid and ortho-phosphoric acid, (c) an alcohol and/or an ester and (d) a peroxy acid and/or a salt thereof are mixed together at a temperature in the range of from 283 to 368 K, the components(a) and (b) being present in concentrations in the range of from 10 to 65 wt. %, with respect to the total weight of the composition and the proportions of components (a) to (d), temperatures, time and intensity of mixing being so selected as to obtain a product in the form of a visually homogeneous, liquid suspension or a visually homogeneous paste, for use as a catalyst in an alkoxylation process.

In yet another embodiment of the present invention, there is provided an alkoxylation process for the preparation of narrow-range alkoxylates, wherein said process comprises contacting and reacting one or more alkylene oxide reactants with one or more carboxylic acid esters, in the presence of a catalytically effective amount of said catalyst composition.

## Detailed Description of the Invention

Alkaline earth metal salts (a) that may be conveniently used in the catalyst composition include calcium and magnesium salts of carboxylic and/or hydroxycarboxylic acids and/or hydrates of the former.

Salts that may be conveniently used as component (a) in the catalyst composition of the present invention are salts of low molecular weight carboxylic acids and/or hydroxycarboxylic acids, that is to say, carboxylic acids and/or hydroxycarboxylic acids having in the range of from 1 to 18 carbon atoms.

Preferred salts are salts of carboxylic acids having in the range of from 1 to 7 carbon atoms and/or salts of hydroxycarboxylic acids having in the range of from 2 to 7 carbon atoms.

More preferred salts are salts of carboxylic acids having in the range of from 2 to 4 carbon atoms and/or salts of hydroxycarboxylic acids having in the range of from 2 to 4 carbon atoms.

Examples of salts that may be used as component (a) of the catalyst composition of the present invention include salts of formic acid, acetic acid, propionic acid, lactic acid, isobutyric acid, 2-hydroxy-2-methylpropanoic acid and benzoic acid.

Calcium salts of carboxylic and/or hydroxycarboxylic acids having in the range of from 1 to 18 carbon atoms, preferably in the range of from 1 to 7 carbon atoms, more preferably in the range of from 2 to 4 carbon atoms and/or hydrates of the former are preferred.

Examples of such calcium salts include calcium acetate and/or calcium lactate and/or hydrates of the former.

Specific examples of oxy-acids that may be used are concentrated (85 %) ortho-phosphoric acid and concentrated (95-97 %) sulphuric acid.

The alcohol which may be used as component (c) in the catalyst composition of the present invention may be a primary, secondary or tertiary alcohol.

The alcohol and/or an ester used as component (c) in the catalyst composition of the present invention is preferably an alcohol having in the range of from 1 to 6 carbon atoms and/or a carboxylic acid ester having in the range of from 2 to 39 carbon atoms. In a particularly preferred embodiment of the present invention, the ester component (c) may be alkoxylated.

Examples of alcohols and esters that may be conveniently used as component (c) of the catalyst composition of the present invention include methanol, ethanol, propanol, 2-propanol (isopropyl alcohol), butanol, 2-butanol (*sec*-butyl alcohol) and 2-methyl-2-propanol (*tert*-butyl alcohol), pentanol, 2-pentanol, 3-pentanol, 3-methyl-1-butanol (isoamyl alcohol), 2,2-dimethyl-1-propanol (neopentyl alcohol), 2-methyl-2-butanol (*tert*-amyl alcohol), hexanol, 2-hexanol, 3-hexanol, methyl formate, ethyl formate, propyl formate, isopropyl formate, butyl formate, sec-butyl formate, isobutyl formate, pentyl formate, isoamyl formate, hexyl formate, cyclohexyl formate, heptyl formate, benzyl formate, octyl formate, nonyl formate, 1-decyl formate, the formic acid esters of *sec*-decyl alcohols, such as 2-decanol, 3-decanol, 4-decanol and 5-decanol and their mixtures, the formic acid esters of a mixture of *sec*-undecyl alcohols and *sec*-dodecyl alcohols, 1-dodecyl formate, formic acid esters of *sec*-dodecyl alcohols, such as 2-dodecanol, 3-dodecanol, 4-dodecanol, 5-dodecanol and 6-dodecanol and their mixtures, formic acid esters of a mixture of *sec*-tridecyl alcohols and *sec*-tetradecyl alcohols, the formic acid esters of a mixture of linear or branched *sec*-pentadecyl alcohols and *sec*-hexadecyl alcohols or a mixture of linear or branched *sec*-pentadecyl alcohols, *sec*-hexadecyl alcohols, *sec*-heptadecyl alcohols and *sec*-octadecyl alcohols, formic acid esters of a mixture of *sec*-nonadecyl alcohols, *sec*-eicosyl alcohols, *sec*-heneicosyl alcohols, *sec*-docosyl alcohols, *sec*-tricosyl alcohols, *sec*-tetracosyl alcohols, *sec*-pentacosyl alcohols, *sec*-hexacosyl alcohols, *sec*-heptacosyl alcohols, *sec*-octacosyl alcohols, *sec*-nonacosyl alcohols, *sec*-triacontyl alcohols, *sec*-hentriacontyl alcohols or *sec*-dotriacontyl alcohols, methyl acetate, ethyl acetate, propyl acetate, isopropyl acetate, butyl acetate, *sec*-butyl acetate, isobutyl acetate, *tert*-butyl acetate, pentyl acetate, 2-pentyl acetate, 3-pentyl acetate, isoamyl acetate, *tert*-amyl acetate, hexyl acetate, cyclohexyl acetate, heptyl acetate, benzyl acetate, octyl acetate, nonyl acetate, decyl acetate, the acetic acid esters of *sec*-decyl alcohols, such as 2-decanol, 3-decanol, 4-decanol and 5-decanol and their mixtures, the acetic acid esters of a mixture of *sec*-undecyl alcohols and *sec*-dodecyl alcohols, 1-dodecyl acetate, acetic acid esters of *sec*-dodecyl alcohols, such as 2-dodecanol, 3-dodecanol, 4-dodecanol, 5-dodecanol and 6-dodecanol and their mixtures, acetic acid esters of a mixture of *sec*-tridecyl alcohols and *sec*-tetradecyl alcohols, the acetic acid esters of a mixture of linear or branched *sec*-pentadecyl alcohols and *sec*-hexadecyl alcohols or a mixture of linear or branched *sec*-pentadecyl alcohols, *sec*-hexadecyl alcohols, *sec*-heptadecyl alcohols and *sec*-octadecyl alcohols, acetic acid esters of a mixture of *sec*-nonadecyl alcohols, *sec*-eicosyl alcohols, *sec*-heneicosyl alcohols, *sec*-docosyl alcohols, *sec*-tricosyl alcohols, *sec*-tetracosyl alcohols, *sec*-pentacosyl alcohols, *sec*-hexacosyl alcohols, *sec*-heptacosyl alcohols, *sec*-octacosyl alcohols, *sec*-nonacosyl alcohols, *sec*-triacontyl alcohols, *sec*-hentriacontyl alcohols, or *sec*-dotriacontyl alcohols, methyl isobutyrate, ethyl isobutyrate, propyl isobutyrate, isopropyl isobutyrate, butyl isobutyrate, *sec*-butyl isobutyrate, isobutyl isobutyrate, *tert*-butyl isobutyrate, pentyl isobutyrate, 2-pentyl isobutyrate, 3-pentyl isobutyrate, isoamyl isobutyrate, *tert*-amyl isobutyrate, hexyl isobutyrate, cyclohexyl isobutyrate, heptyl isobutyrate, benzyl isobutyrate, octyl isobutyrate, nonyl isobutyrate, decyl isobutyrate, the isobutyric acid esters of *sec*-decyl alcohols, such as 2-decanol, 3-decanol, 4-decanol and 5-decanol and their mixtures, the isobutyric acid esters of a mixture of *sec*-undecyl alcohols and *sec*-dodecyl alcohols, 1-dodecyl isobutyrate, isobutyric acid esters of *sec*-dodecyl alcohols, such as 2-dodecanol, 3-dodecanol, 4-dodecanol, 5-dodecanol and 6-dodecanol and their mixtures, isobutyric acid esters of a mixture of *sec*-tridecyl alcohols and *sec*-tetradecyl alcohols, the isobutyric acid esters of a mixture of linear or branched *sec*-pentadecyl alcohols and *sec*-hexadecyl alcohols or a mixture of linear or branched *sec*-pentadecyl alcohols, *sec*-hexadecyl alcohols, *sec*-heptadecyl alcohols and *sec*-octadecyl alcohols, isobutyric acid esters of a mixture of *sec*-nonadecyl alcohols, *sec*-eicosyl alcohols, *sec*-heneicosyl alcohols, *sec*-docosyl alcohols, *sec*-tricosyl alcohols, *sec*-tetracosyl alcohols, *sec*-pentacosyl alcohols, *sec*-hexacosyl alcohols, *sec*-heptacosyl alcohols, *sec*-octacosyl alcohols, *sec*-nonacosyl alcohols, *sec*-triacontyl alcohols, *sec*-hentriacontyl alcohols, or *sec*-dotriacontyl alcohols, methyl benzoate, ethyl benzoate, propyl benzoate, isopropyl benzoate, butyl benzoate, *sec*-butyl benzoate, isobutyl benzoate, *tert*-butyl benzoate, pentyl benzoate, 2-pentyl benzoate, 3-pentyl benzoate, isoamyl benzoate, *tert*-amyl benzoate, hexyl benzoate, cyclohexyl benzoate, heptyl benzoate, benzyl benzoate, octyl benzoate, nonyl benzoate, decyl benzoate, the benzoic

acid esters of *sec*-decyl alcohols, such as 2-decanol, 3-decanol, 4-decanol and 5-decanol and their mixtures, the benzoic acid esters of a mixture of *sec*-undecyl alcohols and *sec*-dodecyl alcohols, 1-dodecyl benzoate, benzoic acid esters of *sec*-dodecyl alcohols, such as 2-dodecanol, 3-dodecanol, 4-dodecanol, 5-dodecanol and 6-dodecanol and their mixtures, benzoic acid esters of a mixture of *sec*-tridecyl alcohols and *sec*-tetradecyl alcohols, the benzoic acid esters of a mixture of linear or branched *sec*-pentadecyl alcohols and *sec*-hexadecyl alcohols or a mixture of linear or branched *sec*-pentadecyl alcohols, *sec*-hexadecyl alcohols, *sec*-heptadecyl alcohols and *sec*-octadecyl alcohols, benzoic acid esters of a mixture of *sec*-nonadecyl alcohols, *sec*-eicosyl alcohols, *sec*-heneicosyl alcohols, *sec*-docosyl alcohols, *sec*-tricosyl alcohols, *sec*-tetracosyl alcohols, *sec*-pentacosyl alcohols, *sec*-hexacosyl alcohols, *sec*-heptacosyl alcohols, *sec*-octacosyl alcohols, *sec*-nonacosyl alcohols, *sec*-triacontyl alcohols, *sec*-hentriacontyl alcohols or *sec*-dotriacontyl alcohols, methyl caproate, ethyl caproate, isopropyl caproate, methyl caprylate, ethyl caprylate, isopropyl caprylate, methyl caprate, ethyl caprate, isopropyl caprate, methyl laurate, ethyl laurate, isopropyl laurate, methyl myristate, ethyl myristate, isopropyl myristate, methyl palmitate, ethyl palmitate, isopropyl palmitate, methyl stearate, ethyl stearate, isopropyl stearate, methyl oleate, ethyl oleate isopropyl oleate, methyl linoleate, ethyl linoleate isopropyl linoleate, methyl linolenate, ethyl linolenate isopropyl linolenate, methyl arachidate, ethyl arachidate, isopropyl arachidate, methyl behenate, ethyl behenate, isopropyl behenate and mixtures thereof.

In one embodiment of the present invention, the catalyst composition comprises an ester as component (c) which is the same as the carboxylic acid ester being alkoxylated.

In a preferred embodiment, the catalyst composition of the present invention may comprise:

(a) one or more alkaline earth metal salts of carboxylic and/or hydroxycarboxylic acids and/or hydrates of the former in an amount in the range of from 8 to 53 wt. %, more preferably in the range of from 16 to 26 wt. %, based on the total weight of the catalyst composition;

(b) an oxy-acid selected from sulphuric acid and ortho-phosphoric acid in an amount in the range of from 2 to 13 wt. %, more preferably in the range of from 4 to 7 wt. %, based on the total weight of the catalyst composition;

(c) an alcohol and/or an ester in an amount in the range of from 34 to 90 wt. %, more preferably in the range of from 67 to 80 wt. %, based on the total weight of the catalyst composition; and

(d) a peroxy acid and/or a salt thereof in an amount in the range of from 10 to 10000 ppm (wt./wt.), more preferably in the range of from 30 to 3000 ppm (wt./wt.) and most preferably in the range of from 100 to 1000 ppm (wt./wt.), based on the total weight of the catalyst composition

and/or products of the reciprocal reactions of (a), (b), (c) and/or (d).

It will be appreciated that the exact amounts of components (a)-(d) are selected from the above ranges such that the amounts thereof total 100 wt. %.

The peroxy acid used as component (d) in the catalyst composition of the present invention may be conveniently selected from percarboxylic acid, perhalic acid, hypohalous acid, percarbonic acid, perboric acid, perphosphoric acid, persulphuric acid and mixtures thereof.

Peroxy acid salts that may be conveniently used include ammonium, alkali metal and/or alkaline earth metal salts. Examples of alkali metals and alkaline earth metal salts that are preferred include sodium, potassium, calcium, magnesium and barium salts.

Examples of particularly preferred peroxy acid salts are ammonium, alkali metal and alkaline earth metal persulphates, in particular ammonium, sodium, potassium and barium persulphates. Ammonium persulphate (also known as ammonium peroxydisulphate, $(NH_4)_2SO_8$) is preferred. Such salts are commercially available.

In a preferred embodiment of the present invention, the alkoxylation catalyst composition is in the form of a visually homogeneous, liquid suspension or a homogeneous paste.

In one embodiment of the present invention, the catalyst composition may be used as a freshly-prepared finely milled (for example, in a colloid mill, in a ball-mill or with an ultrasonic homogeniser, such as the "Labsonic P" of 400 W max, manufactured by Sartorius AG, Goettingen, Germany) mixture of components (a) to (d).

The catalyst composition of the present invention is preferably prepared by firstly admixing a peroxy acid and/or a salt thereof to the oxy-acid to give a peroxy-containing acidic solution comprising a peroxy acid and/or a peroxy salt in an amount in the range of from 0.02 to 20 wt. %, more preferably in the range of from 0.06 to 6 wt. %, and most preferably in the range of from 0.2 to 2 wt. %, based on the total weight of the peroxy acid and/or a salt thereof and the oxy-acid.

This peroxy-containing acidic solution is admixed slowly under vigorous stirring to a suspension of one or more alkaline earth metal salts of carboxylic and/or hydroxycarboxylic acids and/or hydrates of the former in an alcohol and/or an ester at a temperature typically in the range of from 283 to 368 K, preferably at temperatures below 313 K.

For example, in a preferred embodiment, a 0.55 wt. % solution of ammonium peroxydisulphate in concentrated (95-97 %) sulphuric acid may be admixed with a suspension of calcium acetate monohydrate or calcium lactate under vigorous stirring at such a rate that the temperature does not rise above 313 K, even more preferably at a rate such that

the temperature is below 303 K.

In a particularly preferred embodiment, a 0.55 wt. % solution of ammonium peroxydisulphate in concentrated (95-97 %) sulphuric acid may be admixed with a suspension of calcium acetate monohydrate under vigorous stirring at such a rate that the temperature does not rise above 303 K, whereupon the suspension is subsequently treated with an ultrasonic mixing device at temperatures below 303 K for 1 minute.

In a particularly preferred embodiment of the present invention, said catalyst composition comprises (a) one or more alkaline earth metal salts of carboxylic and/or hydroxycarboxylic acids and/or hydrates of the former, (b) an oxy-acid selected from sulphuric acid and ortho-phosphoric acid, (c) an alcohol and/or an ester and (d) a peroxy acid and/or a salt thereof, and/or products of the reciprocal reactions of (a), (b), (c) and/or (d), being formed on homogenisation, the components (a) and (b) being present in concentrations in the range of from 10 to 65 wt. %, with respect to the total weight of the catalyst composition, as calculated from the proportions of components (a) to (d) employed in the catalyst preparation.

The catalyst composition of the present invention may be used in the reaction mixture of the alkoxylation process of the present invention in a catalytically effective amount, i.e. an amount sufficient to promote the alkoxylation reaction or influence the alkylene oxide adduct distribution of the product. Although a specific quantity of catalyst is not critical to the process of the present invention, preference may be expressed for use of the catalyst composition in an amount of at least 0.05 wt. %, while an amount in the range of from 0.2 to 5 wt. % is more preferred and an amount in the range of from 0.5 to 2 wt. % is most preferred for typical embodiments. These percentages are in terms of the weight of the catalyst composition in the process mixture relative to the weight of fatty acid ester in that mixture.

Substantially greater quantities of the catalyst composition of the present invention may also be employed, e.g. up to 10 wt. %, or more. As a rule, the higher the desired average alkylene oxide adduct number of the alkoxylate product and the higher the desired rate of reaction, then the greater the required quantity of catalyst.

In the alkoxylation process of the present invention, the alkylene oxide (epoxide) reactants may be conveniently selected from one or more vicinal alkylene oxides, particularly the lower alkylene oxides and more particularly those in the $C_2$ to $C_4$ range. In general, the alkylene oxides may be represented by the formula,

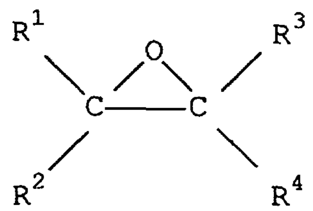

wherein each of the $R^1$, $R^2$, $R^3$ and $R^4$ moieties is individually selected from the group consisting of hydrogen and alkyl moieties. Reactants which comprise ethylene oxide, propylene oxide, or mixtures of ethylene oxide and propylene oxide are more preferred, particularly those which consist essentially of ethylene oxide and propylene oxide. Alkylene oxide reactants consisting essentially of ethylene oxide are considered most preferred from the standpoint of commercial opportunities for the practice of alkoxylation processes, and also from the standpoint of the preparation of products having narrow-range ethylene oxide adduct distributions.

Carboxylic acid esters that may be conveniently employed in the alkoxylation process of the present invention include esters of formic acid, acetic acid, propionic acid, butyric acid, isobutyric acid, pentanoic acid, caproic acid and optionally substituted benzoic acids.

Carboxylic acid esters that may be conveniently employed in the alkoxylation process of the present invention are preferably fatty acid esters, more preferably fatty acid esters having in the range of from 7 to 39 carbon atoms.

Examples of said fatty acid esters include esters of caproic acid, caprylic acid, capric acid, lauric acid, myristic acid, palmitic acid, stearic acid, oleic acid, linoleic acid, linolenic acid, arachidic acid, behenic acid and mixtures thereof.

Carboxylic acid esters, and in particular fatty acid esters, that may be conveniently employed in the alkoxylation process of the present invention include, for example, methyl esters, ethyl esters, isopropyl esters, isobutyl esters, *sec*-butyl esters and *tert*-butyl esters, amyl esters, *sec*-amyl esters, isoamyl esters, neopentyl esters and *tert*-amyl esters, *sec*-decyl esters such as 2-decyl, 3-decyl, 4-decyl and 5-decyl esters and their mixtures, *sec*-undecyl esters, *sec*-dodecyl esters, such as 2-undecyl, 3-undecyl, 4-undecyl, 5-undecyl, 2-dodecyl, 3-dodecyl, 4-dodecyl, 5-dodecyl and 6-dodecyl esters and their mixtures, *sec*-tridecyl esters and *sec*-tetradecyl esters, such as 2-tridecyl, 3-tridecyl, 4-tridecyl, 5-tridecyl, 6-tridecyl, 2-tetradecyl, 3-tetradecyl, 4-tetradecyl, 5-tetradecyl, 6-tetradecyl, 7-tetradecyl esters and their mixtures, *sec*-pentadecyl esters, *sec*-hexadecyl esters, *sec*-heptadecyl esters, *sec*-octadecyl esters and their mixtures, *sec*-nonadecyl esters, *sec*-eicosyl esters, *sec*-heneicosyl esters, *sec*-docosyl esters, *sec*-tricosyl esters, *sec*-tetracosyl esters, *sec*-pentacosyl esters, *sec*-hexacosyl esters, *sec*-heptacosyl esters, *sec*-octacosyl esters, *sec*-nonacosyl esters, *sec*-triacontyl esters, *sec*-hentriacontyl esters, *sec*-dotriacontyl esters and their mixtures.

Preferred carboxylic acid esters that may be conveniently employed in the alkoxylation process of the present invention include methyl, ethyl, isopropyl, isobutyl, *sec*-butyl, *tert*-butyl, amyl, *sec*-amyl, isoamyl, neopentyl, *tert*-amyl, *sec*-decyl such as 2-decyl, 3-decyl, 4-decyl and 5-decyl, *sec*-undecyl, *sec*-dodecyl such as 2-undecyl, 3-undecyl, 4-undecyl, 5-undecyl, 2-dodecyl, 3-dodecyl, 4-dodecyl, 5-dodecyl and 6-dodecyl, *sec*-tridecyl, *sec*-tetradecyl such as 2-tridecyl, 3-tridecyl, 4-tridecyl, 5-tridecyl, 6-tridecyl, 2-tetradecyl, 3-tetradecyl, 4-tetradecyl, 5-tetradecyl, 6-tetradecyl, 7-tetradecyl, *sec*-pentadecyl, *sec*-hexadecyl, *sec*-heptadecyl, *sec*-octadecyl, *sec*-nonadecyl, *sec*-eicosyl, *sec*-heneicosyl, *sec*-docosyl, *sec*-tricosyl, *sec*-tetracosyl, *sec*-pentacosyl, *sec*-hexacosyl, *sec*-heptacosyl, *sec*-octacosyl, *sec*-nonacosyl, *sec*-triacontyl, *sec*-hentriacontyl, and *sec*-dotriacontyl acetates, and mixtures thereof.

Another class of preferred carboxylic acid esters that may be conveniently employed in the alkoxylation process of the present invention include methyl, ethyl, isopropyl, isobutyl, *sec*-butyl, *tert*-butyl, amyl, *sec*-amyl, isoamyl, neopentyl, *tert*-amyl, *sec*-decyl such as 2-decyl, 3-decyl, 4-decyl and 5-decyl, *sec*-undecyl, *sec*-dodecyl such as 2-undecyl, 3-undecyl, 4-undecyl, 5-undecyl, 2-dodecyl, 3-dodecyl, 4-dodecyl, 5-dodecyl and 6-dodecyl, *sec*-tridecyl, *sec*-tetradecyl such as 2-tridecyl, 3-tridecyl, 4-tridecyl, 5-tridecyl, 6-tridecyl, 2-tetradecyl, 3-tetradecyl, 4-tetradecyl, 5-tetradecyl, 6-tetradecyl, 7-tetradecyl, *sec*-pentadecyl, *sec*-hexadecyl, *sec*-heptadecyl, *sec*-octadecyl, *sec*-nonadecyl, sec-eicosyl, *sec*-heneicosyl, *sec*-docosyl, *sec*-tricosyl, *sec*-tetracosyl, *sec*-pentacosyl, *sec*-hexacosyl, *sec*-heptacosyl, *sec*-octacosyl, *sec*-nonacosyl, *sec*-triacontyl, *sec*-hentriacontyl and *sec*-dotriacontyl isobutyrates, and mixtures thereof.

Yet another class of preferred carboxylic acid esters that may be conveniently employed in the alkoxylation process of the present invention include methyl, ethyl, isopropyl, isobutyl, *sec*-butyl, *tert*-butyl, amyl, *sec*-amyl, isoamyl, neopentyl, *tert*-amyl, *sec*-decyl such as 2-decyl, 3-decyl, 4-decyl and 5-decyl, *sec*-undecyl, *sec*-dodecyl such as 2-undecyl, 3-undecyl, 4-undecyl, 5-undecyl, 2-dodecyl, 3-dodecyl, 4-dodecyl, 5-dodecyl and 6-dodecyl, *sec*-tridecyl, *sec*-tetradecyl such as 2-tridecyl, 3-tridecyl, 4-tridecyl, 5-tridecyl, 6-tridecyl, 2-tetradecyl, 3-tetradecyl, 4-tetradecyl, 5-tetradecyl, 6-tetradecyl, 7-tetradecyl, *sec*-pentadecyl, *sec*-hexadecyl, *sec*-heptadecyl, *sec*-octadecyl, *sec*-nonadecyl, *sec*-eicosyl, *sec*-heneicosyl, *sec*-docosyl, *sec*-tricosyl, *sec*-tetracosyl, *sec*-pentacosyl, *sec*-hexacosyl, *sec*-heptacosyl, *sec*-octacosyl, *sec*-nonacosyl, *sec*-triacontyl, *sec*-hentriacontyl and *sec*-dotriacontyl benzoates, and mixtures thereof.

In terms of processing procedures, the alkoxylation reaction in the process of the present invention may be conducted in a generally conventional manner. For example, the catalyst composition in the liquid carboxylic acid ester reactant may be contacted, preferably under agitation, with alkylene oxide reactant, which is typically introduced in gaseous form, at least for the lower alkylene oxides.

Additional liquid carboxylic acid ester reactant can optionally be added anytime before the addition of the alkylene oxide reactant. Thus, a concentrated catalyst reaction mixture can be made and a portion can be used as necessary.

In preferred embodiments of the process of the present invention, the alkylene oxide reactant is ethylene oxide or propylene oxide or a mixture of ethylene oxide and propylene oxide.

In a particularly preferred embodiment, ethylene oxide is contacted and reacted with a carboxylic acid ester, preferably a fatty acid ester, in the presence of a catalytically effective amount of a alkoxylation catalyst composition as described herein. Additional liquid carboxylic acid ester reactant can be optionally added at any time during the process.

In another embodiment of the present invention, the alkoxylation process may be conducted according to the method described in WO-A-2005/115964.

That is to say, in one embodiment, the alkoxylation process of the present invention comprises introducing the alkylene oxide reactant into a reactor filled with the carboxylic acid ester reactant in the presence of a catalyst composition as described herein, wherein the reactor is filled with a portion (x') of a pre-designed total quantity (X) of the carboxylic acid ester reactant to be subjected to alkoxylation and with a total pre-designed quantity (Y) of said catalyst composition, whereupon another portion (z') of the pre-designed total quantity (Z) of alkylene oxide reactant is fed to the reactor to activate said catalyst composition and induce a reaction and after the introduced quantity (z') of alkylene oxide reactant has been converted entirely or partly, the carboxylic acid ester reactant feed is supplemented to the pre-designed quantity (X) and alkylene oxide reactant is continued to be fed until the pre-designed quantity (Z) has been introduced, preferably, the portion (x') of the carboxylic acid ester reactant introduced in the initial phase of the synthesis is as small as possible relative to the total pre-designed carboxylic acid ester reactant feed (X), that is, its quantity is around the minimum feed specified for a given reactor, that is, when the value of the quotient x'/X is as small as possible.

While the above procedures describe batch modes of operation, the present invention is equally applicable to a continuous process.

Overall, the two reactants are utilised in quantities which are predetermined to yield an alkoxylate product of the desired mean or average adduct number. The average adduct number of the product is not critical to this process. Such products commonly have an average adduct number in the range of from less than 1 to 30, or greater.

In general terms, suitable and preferred process temperatures and pressures for purposes of process of the present invention are the same as in conventional alkoxylation reaction between the same reactants, employing conventional catalysts. A temperature of at least 90 °C, particularly at least 120 °C, and most particularly at least 130 °C,

is typically preferred from the standpoint of the rate of reaction, while a temperature of less than 250 °C, particularly of less than 210 °C, and most particularly of less than 190 °C, is typically desirable to minimise degradation of the product. As is known in the art, the process temperature can be optimised for given reactants, taking such factors into account.

Super-atmospheric pressures, e.g. pressures in the range of from 0.7 and 1 MPa gauge (about 10 and about 150 psig), are preferred, with pressure being sufficient to maintain the carboxylic acid ester reactant substantially in the liquid state.

When the carboxylic acid ester reactant is a liquid and the alkylene oxide reactant is a vapour, alkoxylation is then suitably conducted by introducing alkylene oxide into a pressure reactor containing the liquid carboxylic acid ester reactant and the catalyst composition. For considerations of process safety the alkylene oxide reactant is preferably diluted with an inert gas such as nitrogen, for instance, to a vapour phase concentration of about 50 vol. percent or less. The reaction can, however, be safely accomplished at greater alkylene oxide concentrations, greater total pressure and greater partial pressure of alkylene oxide if suitable precautions, known in the art, are taken to manage the risks of explosion.

The time required to complete an alkoxylation process according to the present invention is dependent both upon the degree of alkoxylation that is desired (i.e. upon the average alkylene oxide adduct number of the product) as well as upon the rate of the alkoxylation reaction (which is, in turn, dependent upon temperature, catalyst quantity and nature of the reactants). A typical reaction time for preferred embodiments, particularly for when the alkylene oxide is gaseous, is less than 12 hours. When ethylene oxide is used as the alkylene oxide, the typical reaction time is less than 5 hours. When propylene oxide is used as the alkylene oxide, the typical reaction time is less than 8 hours.

After the alkoxylation reaction has been completed, the product is preferably cooled. If desired, the catalyst composition can be removed from the final product, although catalyst removal is not necessary to the process of the present invention. Catalyst residues may be removed, for example, by filtration, precipitation or extraction. A number of specific chemical and physical treatment methods have been found to facilitate removal of catalyst residues from a liquid product. Such treatments include contact of the alkoxylation product with strong acids such as phosphoric and/or oxalic acids or with solid organic acids such as those sold under the trade designations "NAFION" H+ (E.I. du Pont de Nemours & co., Inc.) and "AMBERLITE" IR 120H (Rohm & Haas Co.) from Sigma-Aldrich; contact with alkali metal carbonates and bicarbonates; contact with zeolites such as type Y zeolite or mordenite; or contact with certain clays. Typically, such treatments are followed by filtration or precipitation of the solids from the product. In many cases filtration, precipitation or centrifugation is most efficient at elevated temperature.

Preferred carboxylic acid ester alkoxylate compositions, in particular fatty ester alkoxylate compositions, which may be prepared by the present process, are those comprising one or more alkoxylated carboxylic acid esters, in particular alkoxylated fatty esters, having the formula (I):

$$R^1\text{-C(O) - (OA)}_n\text{-OR}^2 \qquad \text{(I)}$$

wherein $R^1$ is a straight chain or branched alkyl group having from 1 to 30 carbon atoms, an optionally substituted cycloalkyl group having 5 to 30 carbon atoms or an optionally substituted aryl group having 6 to 30 carbon atoms, OA represents one or more oxyalkylene moieties which may be the same or different, n is an integer in the range of from 0 to 70 and $R^2$ is a straight chain or branched alkyl group having from 1 to 32 carbon atoms, an optionally substituted cycloalkyl group of 5 to 32 carbon atoms or an optionally substituted bicycloalkyl group of 7 to 32 carbon atoms.

Particularly preferred carboxylic acid ester alkoxylates, including fatty ester alkoxylates, that may be produced by the process of the present invention are those wherein $R^1$ is a straight chain or branched alkyl group having from 1 to 22 carbon atoms or an optionally substituted phenyl group, OA is selected independently from oxyethylene and oxypropylene moieties, n is an integer in the range of from 1 to 30 and $R^2$ is a straight chain or branched alkyl group having from 1 to 32 carbon atoms.

Carboxylic acid ester alkoxylates produced by the alkoxylation process of the present invention which are especially preferred for the purpose of detergent applications are compounds of formula (I) wherein $R^1$ is an optionally substituted phenyl group, preferably a phenyl group, hydrogen or an alkyl group having in the range of from 1 to 22 carbon atoms, preferably 1 carbon atom, OA is selected independently from oxyethylene and oxypropylene moieties, n is an integer in the range of from 1 to 30 and $R^2$ is a straight chain or branched alkyl group having in the range of from 1 to 32 carbon atoms.

Fatty acid ester alkoxylates produced by the alkoxylation process of the present invention which are especially preferred for the purpose of detergent applications are compounds of formula (I), wherein $R^1$ is an alkyl group having from 6 to 22 carbon atoms, preferably from 9 to 15 carbon atoms, OA is selected independently from oxyethylene and oxypropylene moieties, n is an integer in the range of from 1 to 30 and $R^2$ is a straight chain or branched alkyl group having from 1 to 32 carbon atoms, preferably from 1 to 6 carbon atoms, most preferably from 1 to 3 carbon atoms.

In formula (I), the different OA groups can be distributed randomly along the alkoxide chain or be present as block (co)-polymers.

The catalyst composition of the present invention surprisingly allows for the production of carboxylic acid ester alkoxylates, preferably fatty ester alkoxylates and more preferably narrow range fatty ester alkoxylates, which have advantageous colour properties, in particular having reduced yellowing.

The present invention will now be illustrated by the following examples which are not intended to limit the scope of the invention in any way.

Examples

Catalyst Preparation 1

Calcium acetate monohydrate (5.5 g) (as 99 % grade available from Aldrich) was added to 23 ml isopropyl alcohol (IPA, as PA-grade available from Merck) at ambient temperature and atmospheric pressure. About 0.8 ml of concentrated sulphuric acid (95-97% available from Merck) to which 0.55 wt. % ammonium persulphate (available from Aldrich) has been added, was dosed to the mixture under vigorous stirring with a magnetic bar at such a rate that the temperature remains below 40 °C. After that the mixture, a milky suspension, was stirred for another 30 minutes and was thereafter used as such as ethylene oxide (EO) insertion catalyst.

Catalyst Preparation 2 (Comparative)

This catalyst preparation was carried out almost identical to catalyst preparation 1 with the only difference that now just concentrated sulphuric acid (95-97 % available from Merck) was dosed.

Ethoxylation Examples 1 and 2

Methyl laurate (40 g) (available in > 97 % purity from Fluka A.G. Switzerland) and 0.8 g catalyst suspension (catalyst 1 or comparative catalyst 2) were added to a 120 ml autoclave (stainless steel). After closing, the autoclave was pressurized 3 times with nitrogen (4-5 barg) to purge the gas cap and it was heated to 130 °C. At 130 °C the autoclave was purged with nitrogen (10-15 litre/min) for 30 minutes to dry the contents. The autoclave was then pressurized with 5 barg nitrogen and it was further heated to 165 °C.

At 165 °C ethylene oxide was added in portions with the use of an ISCO pump. For safety reasons the amount of each EO-addition should not give a higher concentration than 50 %v/v in nitrogen (generally each portion added consisted of 2-3 g of ethylene oxide). After addition of the first portion of ethylene oxide a short induction period of ca. 5 minutes was observed. Then the reaction started and the autoclave-pressure decreased quickly. When the pressure decrease levelled off (after 15-20 minutes), the next portion of ethylene oxide was added. The additions were repeated until the desired total amount of ethylene oxide was reached.

When all ethylene oxide was added, 30 minutes extra reaction time was allowed to let the remaining EO react away. The autoclave was then cooled down to 80 °C and at this temperature the autoclave content was purged with nitrogen for 20 minutes to remove the last traces of dissolved free ethylene oxide before opening the autoclave. The temperature was then decreased to room temperature to enable a safe discharge of the product. The details of these ethoxylation examples have been summarised in Table 1.

The average EO-number and the EO-distribution of the products were determined by GC (see Table 2, Table 3 and Figure 1). The conversion of the methyl ester and the average EO-number were determined by NMR.

Table 1

| Ex. | Catalyst Preparation (g) | | | Ammonium persulphate | Catalyst | | | Feed composition | FAME | EO or ethoxylate | Product |
|---|---|---|---|---|---|---|---|---|---|---|---|
| | $Ca(OAc)_2 .H_20$ | IPA | $H_2SO_4$ | (0.55 wt. % in $H_2SO_4$) | Total (g) | Intake (g) | Intake (mol Ca) | | Intake (g) | Intake (g) | Mass (g) |
| 1 | 5.51 | 18.87 | 1.45 | yes | 25.83 | 0.81 | 9.81E-04 | methyllaurate + EO | 40.01 | 39.5 | 78.27 |
| 2 | 5.52 | 18.02 | 1.38 | no | 24.92 | 0.81 | 1.01E-03 | methyllaurate + EO | 40.04 | 39.0 | 78.82 |
| 3 | p-TSA | - | - | - | - | 0.5 | | methyllaurate + PEG-methylether-350 | 100 | 165 | 250 |
| 4 | KOtBu | - | - | - | - | 0.5 | | methyllaurate + PEG-methylether-350 | 50 | 94 | 133 |
| Conditions: 165 °C, 10-25 barg, 120-ml autoclave, 1000 rpm | | | | | | | | | | | |

Table 2

| Ex. | Intake | | | Ethoxylation time (min) | $EO_{av}$ (GC) (unit/ mol) | $EO_{av}$ (NMR) (unit/ mol) | Colour[b] ASTM D1500 | Remarks on colour of MEE-product | 1,4-Dioxane (GC-MS) (ppm wt./wt.) | Acetaldehyde (GC-MS) (ppm wt./wt.) | Ethoxylation rate[c] $\frac{mol\ EO}{mol FAME*h}$ |
|---|---|---|---|---|---|---|---|---|---|---|---|
| | FAME (mol) | EO (mol) | Catalyst (mol Ca) | | | | | | | | |
| 1 | 0.187 | 0.897 | 9.81E-04 | 253 | 4.36 | (4.8)[a] | <0.5 | Slightly hazy water-white liquid | <100 | <100 | 1.03 |
| 2 | 0.187 | 0.885 | 1.01E-03 | 278 | 4.48 | (4.7)[a] | <1.0 | Hazy yellow liquid | <100 | <100 | 0.97 |
| 3 | | | | | | ~7.0 | 2.0 | Clear orange liquid | <100 | 100-200 | |
| 4 | | | | | | ~7.0 | <1.0 | Clear yellow liquid | <100 | <100 | |

[a] EO/FAME intake ratio (mol/mol).
[b] Colour range: 0.5 - 8.0, each step = 0.5; <1.0 means value between 0.5 and 1.0.
[c] Apparent ethoxylation rate from the average EO content of MEE-product determined by GC.

Table 3

| EO-number $(EO)_n$ n= | EO-distribution (%wt./wt.) EXAMPLE 1 | EO-distribution (%wt./wt.) EXAMPLE 2 |
|---|---|---|
| 0 | 3.95 | 4.61 |
| 1 | 2.70 | 2.99 |
| 2 | 7.36 | 7.35 |
| 3 | 13.57 | 12.66 |
| 4 | 18.56 | 15.25 |
| 5 | 17.77 | 15.34 |
| 6 | 13.43 | 13.10 |
| 7 | 8.13 | 9.49 |
| 8 | 4.40 | 6.19 |
| 9 | 2.39 | 3.80 |
| 10 | 1.47 | 2.34 |
| 11 | 1.17 | 1.43 |
| 12 | 1.05 | 1.02 |
| 13 | 0.95 | 0.77 |
| 14 | 0.81 | 0.74 |
| 15 | 0.61 | 0.64 |
| 16 | 0.46 | 0.55 |
| 17 | 0.34 | 0.46 |
| 18 | 0.26 | 0.39 |
| 19 | 0.21 | 0.29 |
| 20 | 0.21 | 0.28 |
| Average EO-number (EO-units/mol) | 4.36 | 4.48 |

Transesterification Examples 3 to 8

Example 3

Methyl laurate (100 g), the methyl ether of polyethylene glycol-350, having an average mol weight of 350 (165 g, available from Across Organics, UK) and the transesterification catalyst p-toluenesulphonic acid monohydrate (0.5 g, available from Aldrich, Belgium) were heated to 120-130 °C in a nitrogen atmosphere during 35 hours. The progress of the conversion was monitored by NMR. The methanol formed was boiled off in vacuo intermittently and finally 250 g of an orange-coloured product resulted (Table 2). NMR analysis of this crude product revealed a conversion of about 75 %.

Example 4

Methyl laurate (50 g), the methyl ether of polyethylene glycol-350 (94 g) and a basic transesterification catalyst, K *tert*-butoxide (0.5 g, available from Aldrich, Belgium), was used, was heated to 90 °C during 15 hours under a reduced pressure (400-100 mbar) to remove the methanol formed during the transesterification in order to force the equilibrium reaction to completion. This resulted in 133 g of a pale-yellow coloured product (ca. 90 % conversion according to NMR; see Table 2).

Example 5

In a similar experiment to example 3, but now employing the above-described calcium acetate/sulphate catalyst 2 as transesterification catalysts gave neither colour formation nor conversion (<5 % by NMR).

Example 6

In a similar experiment to Example 5, but now employing the above-described calcium acetate/sulphate catalyst, which has been prepared by using sulphuric acid containing 0.55 wt. % of ammonium persulphate (catalyst 1), gave again neither colour formation nor conversion (<5 % by NMR).

Example 7

In again a similar experiment to Example 3, but now employing 95-97 % pure sulphuric acid as the transesterification catalyst, gave an even darker orange-coloured product at a similar conversion as in Example 3 (~75 % conversion according to NMR).

Example 8

In a similar experiment to Example 7, but now employing 95-97 % pure sulphuric acid containing 0.55 wt. % ammonium persulphate, as the transesterification catalyst, gave an as dark orange-coloured product at a similar conversion (~75% conversion according to NMR).

Results and Conclusions

From Examples 1 and 2 it is clear that both Ca-catalysts 1 and 2 are effective catalysts for the insertion of EO in the ester bond of methyl laurate. The colours of the respective products of Examples 1 to 4 have been determined by ASTM D1500. This method has been chosen to accommodate colour measurement of the (slightly) hazy products. It is clear that EO insertion into methyl laurate using the Ca-catalyst containing ~300 ppm (wt./wt.) ammonium persulphate (on total Ca-catalyst intake) gives a lower discolouration (yellowing) than without the peroxysulphate (entries 1 and 2 in Tables 1 and 2). The apparent ethoxylation (insertion) rate appears not or hardly affected by the presence of the small amount of ammonium persulphate in the Ca-catalyst (see Table 2). It may be argued that observed slight difference in ethoxylation rate is more likely due to variations in coarseness of the catalyst particles, i.e. variations in surface area. The GC-data indicate that the ethoxylate distribution is more peaked, when the Ca-catalyst containing ammonium persulphate (catalyst 1) is used (Figure 1). This may be beneficial and/or cost-effective (less EO used) for some detergent applications. Under the applied conditions, irrespective of the catalyst used, the amount of unconverted methyl laurate is 4-5 wt. % and the average degree of ethoxylation about 4.4 EO-units per mol. Conversions of approximately 95 % of the methyl laurate are confirmed by NMR data for both catalysts 1 and 2. The presence of ammonium persulphate does not affect the level of noxious by-products such as 1,4-dioxane and acetaldehyde. These by-products are invariably below 100 ppm (wt./wt.) in the MEE product (see Table 2), despite the forcing temperatures and the alleged presence of acid sites in the catalysts during EO-insertion. Only in the MEE product prepared by p-TSA catalysed transesterification an acetaldehyde level of 100-200 ppm (wt./wt.) is observed.

Alternative production of these methylester ethoxylates (MEE) from methyl laurate involves transesterification with polyethylene glycol monomethyl ether with an average molecular weight of 350 (i.e. having about 7 EO units/chain) under acidic or basic conditions (entries 3 and 4 in Tables 1 and 2). The polyethylene glycol methylether has been prepared from methanol by e.g. KOH-catalysed ethoxylation. Despite the less severe transesterification temperature at the expense of an additional reaction step, which undoubtedly leads to a more costly route towards MEE, the colour formation is more severe than with EO-insertion using the Ca-catalyst according to the present invention. This is particularly true, if the p-toluenesulphonic acid catalysed transesterification (entry 3: colour according to ASTM D1500 = 2.0 on a scale of 0.5-8.0) is compared with the methyl ester ethoxylate, which as been prepared according to the present invention (entry 1: colour according to ASTM D1500 <0.5).

The Ca-catalysts 1 and 2 are not effective as transesterification catalysts at least not at 120-130 °C (Examples 5 and 6).

Moreover, the presence of 0.55 wt. % ammonium persulphate in sulphuric acid does not ameliorate the colour formation during sulphuric acid catalysed transesterification (see Examples 7 and 8), not even at 120-130 °C, which is a moderate temperature compared to EO-insertion conditions.

Thus, the afore-mentioned Examples indicate that the addition of a small amount (e.g. 0.55 wt. % on concentrated sulphuric acid, i.e. ~300 ppm (wt./wt.) on total Ca-catalyst) of ammonium persulphate during the preparation of

the Ca-catalyst, composed of isopropyl alcohol, calcium acetate monohydrate and sulphuric acid, gives rise to the formation of a modified catalyst, which upon ethoxylation of a fatty acid methyl ester is effective in suppressing colour-formation at temperatures as high as 165 °C, whilst the ethoxylate distribution shows increased peaking.

The apparent ethoxylation rate is not or hardly affected by the presence of ammonium persulphate in the catalyst.

Accordingly, the present invention provides a novel modified Ca-catalyst and a new catalytic alkoxylation (in particular, ethoxylation) process, whereby renewable fatty acid methyl esters may be efficiently transformed into colourless, biodegradable (due to the "labile" ester bond, which should not hydrolyse during washing or storage), but well-performing non-ionic surfactants.

## Claims

**1.** A catalyst composition for alkoxylation, said catalyst composition comprising,

(a) one or more alkaline earth metal salts of carboxylic and/or hydroxycarboxylic acids and/or hydrates of the former,
(b) an oxy-acid selected from sulphuric acid and ortho-phosphoric acid,
(c) an alcohol and/or an ester,
(d) a peroxy acid and/or a salt thereof

and/or products of the reciprocal reactions of (a), (b), (c) and/or (d).

**2.** Catalyst composition according to Claim 1, wherein the alkaline earth metal salts (a) are selected from calcium salts of carboxylic and/or hydroxycarboxylic acids and/or hydrates of the former.

**3.** Catalyst composition accordingly to Claim 1 or 2, wherein the alkaline earth metal salts (a) are selected from calcium acetate and/or calcium lactate and/or hydrates of the former.

**4.** Catalyst composition according to any one of Claims 1 to 3, wherein the alcohol and/or ester are selected from alcohols having in the range of from 1 to 6 carbon atoms and/or a carboxylic acid esters having in the range of from 2 to 39 carbon atoms.

**5.** Catalyst composition according to any one of Claims 1 to 4, wherein said composition comprises in the range of from 10 to 10,000 ppm (wt./wt.) of a peroxy acid and/or a salt thereof, based on the total weight of the composition.

**6.** Catalyst composition according to any one of Claims 1 to 5, wherein the peroxy acid is selected from percarboxylic acid, perhalic acid, hypohalous acid, percarbonic acid, perboric acid, perphosphoric acid and persulphuric acid.

**7.** Catalyst composition according to any one of Claims 1 to 6, wherein the peroxy acid salt is selected from ammonium, alkali metal and alkaline earth metal salts.

**8.** An alkoxylation process for the preparation of narrow-range alkoxylates, wherein said process comprises contacting and reacting one or more alkylene oxide reactants with one or more carboxylic acid esters, preferably fatty acid esters having in the range of from 7 to 39 carbon atoms, in the presence of a catalytically effective amount of the catalyst composition according to any one of Claims 1 to 7.

**9.** Process according to Claim 8, wherein the alkylene oxide reactant is ethylene oxide, propylene oxide and/or butylene oxide.

**10.** Process according to Claim 8 or 9, wherein the carboxylic acid esters are selected from methyl, ethyl, isopropyl, isobutyl, *sec*-butyl, *tert*-butyl, amyl, *sec*-amyl, isoamyl, neopentyl, *tert*-amyl, *sec*-decyl such as 2-decyl, 3-decyl, 4-decyl and 5-decyl, *sec*-undecyl, *sec*-dodecyl such as 2-undecyl, 3-undecyl, 4-undecyl, 5-undecyl, 2-dodecyl, 3-dodecyl, 4-dodecyl, 5-dodecyl and 6-dodecyl, *sec*-tridecyl, *sec*-tetradecyl such as 2-tridecyl, 3-tridecyl, 4-tridecyl, 5-tridecyl, 6-tridecyl, 2-tetradecyl, 3-tetradecyl, 4-tetradecyl, 5-tetradecyl, 6-tetradecyl, 7-tetradecyl, *sec*-pentadecyl, *sec*-hexadecyl, *sec*-heptadecyl, *sec*-octadecyl, *sec*-nonadecyl, *sec*-eicosyl, *sec*-heneicosyl, *sec*-docosyl, *sec*-tricosyl, *sec*-tetracosyl, *sec*-pentacosyl, *sec*-hexacosyl, *sec*-heptacosyl, *sec*-octacosyl, *sec*-nonacosyl, *sec*-triacontyl, *sec*-hentriacontyl and *sec*-dotriacontyl acetates, and mixtures thereof.

## Patentansprüche

1. Katalysatorzusammensetzung zur Alkoxylierung, welche Katalysatorzusammensetzung

   (a) ein oder mehrere Erdalkalimetallsalze von Carbonsäuren und/oder Hydroxycarbonsäuren und/oder Hydraten der Vorstehenden,
   (b) eine Oxysäure, ausgewählt unter Schwefelsäure und Orthophosphorsäure,
   (c) einen Alkohol und/oder einen Ester,
   (d) eine Peroxysäure und/oder ein Salz hievon,

   und/oder Produkte der wechselseitigen Reaktionen von (a), (b), (c) und/oder (d),
   umfasst.

2. Katalysatorzusammensetzung nach Anspruch 1, wobei die Erdalkalimetallsalze (a) unter Kalziumsalzen von Carbonsäuren und/oder Hydroxycarbonsäuren und/oder Hydraten der Vorstehenden ausgewählt sind.

3. Katalysatorzusammensetzung nach Anspruch 1 oder 2, wobei die Erdalkalimetallsalze (a) unter Kalziumacetat und/oder Kalziumlactat und/oder Hydraten der Vorstehenden ausgewählt sind.

4. Katalysatorzusammensetzung nach einem der Ansprüche 1 bis 3, wobei der Alkohol und/oder der Ester unter Alkoholen mit einem Bereich von 1 bis 6 Kohlenstoffatomen und/oder Carbonsäureestern mit einem Bereich von 2 bis 39 Kohlenstoffatomen ausgewählt sind.

5. Katalysatorzusammensetzung nach einem der Ansprüche 1 bis 4, wobei die Zusammensetzung im Bereich von 10 bis 10.000 ppm (Gew./Gew.) einer Peroxysäure und/oder eines Salzes hievon, basierend auf dem Gesamtgewicht der Zusammensetzung, umfasst.

6. Katalysatorzusammensetzung nach einem der Ansprüche 1 bis 5, wobei die Peroxysäure unter Percarbonsäure, Perhalogensäure, Hypohalogensäure, Percarbonsäure, Perborsäure, Perphosphorsäure und Perschwefelsäure ausgewählt ist.

7. Katalysatorzusammensetzung nach einem der Ansprüche 1 bis 6, wobei das Peroxysäuresalz unter Ammonium-, Alkalimetall- und Erdalkalimetallsalzen ausgewählt ist.

8. Alkoxylierungsverfahren zur Herstellung von einen engen Bereich aufweisenden Alkoxylaten, wobei das Verfahren das In-Kontakt-Bringen und das Umsetzen von einem oder mehreren Alkylenoxidreaktanten mit einem oder mehreren Carbonsäureestern, vorzugsweise Fettsäureestern mit im Bereich von 7 bis 39 Kohlenstoffatomen, in Gegenwart einer katalytisch wirksamen Menge der Katalysatorzusammensetzung nach einem der Ansprüche 1 bis 7 umfasst.

9. Verfahren nach Anspruch 8, wobei der Alkylenoxidreaktant Ethylenoxid, Propylenoxid und/oder Butylenoxid ist.

10. Verfahren nach Anspruch 8 oder 9, wobei die Carbonsäureester unter Methyl-, Ethyl-, Isopropyl-, Isobutyl-, sec-Butyl-, tert-Butyl-, Amyl-, sec-Amyl-, Isoamyl-, Neopentyl-, tert-Amyl-, sec-Decyl-, wie 2-Decyl-, 3-Decyl-, 4-Decyl- und 5-Decyl-, sec-Undecyl-, sec-Dodecyl-, wie 2-Undecyl-, 3-Undecyl-, 4-Undecyl-, 5-Undecyl-, 2-Dodecyl-, 3-Dodecyl-, 4-Dodecyl-, 5-Dodecyl- und 6-Dodecyl, sec-Tridecyl-, sec-Tetradecyl-, wie 2-Tridecyl-, 3-Tridecyl-, 4-Tridecyl-, 5-Tridecyl-, 6-Tridecyl-, 2-Tetradecyl-, 3-Tetradecyl-, 4-Tetradecyl-, 5-Tetradecyl-, 6-Tetradecyl-, 7-Tetradecyl-, sec-Pentadecyl-, sec-Hexadecyl-, sec-Heptadecyl-, sec-Octadecyl-, sec-Nonadecyl-, sec-Eicosyl-, sec-Heneicosyl-, sec-Docosyl-, sec-Tricosyl-, sec-Tetracosyl-, sec-Pentacosyl-, sec-Hexacosyl-, sec-Heptacosyl-, sec-Octacosyl-, sec-Nonacosyl-, sec-Triacontyl-, sec-Hentriacontyl- und sec-Dotriacontylacetaten, und Mischungen hievon ausgewählt sind.

## Revendications

1. Composition de catalyseur pour alcoxylation, ladite composition de catalyseur comprenant,

   (a) un ou plusieurs sels de métaux alcalino-terreux d'acides carboxyliques et/ou hydroxycarboxyliques et/ou

hydrates de ces derniers,
(b) un oxyacide choisi entre l'acide sulfurique et l'acide orthophosphorique,
(c) un alcool et/ou un ester,
(d) un péroxyacide et/ou un sel de celui-ci,

et/ou des produits des réactions inverses de (a), (b), (c) et/ou (d).

**2.** Composition de catalyseur selon la revendication 1, dans laquelle les sels de métaux alcalino-terreux (a) sont choisis parmi des sels de calcium d'acides carboxyliques et/ou hydroxycarboxyliques et/ou hydrates de ces derniers.

**3.** Composition de catalyseur selon la revendication 1 ou 2, dans laquelle les sels de métaux alcalino-terreux (a) sont choisis parmi l'acétate de calcium et/ou le lactate de calcium et/ou des hydrates de ces derniers.

**4.** Composition de catalyseur selon l'une quelconque des revendications 1 à 3, dans laquelle l'alcool et/ou l'ester sont choisis parmi des alcools ayant un nombre d'atomes de carbone dans la plage de 1 à 6 et/ou des esters d'acide carboxylique ayant un nombre d'atomes de carbone dans la plage de 2 à 39.

**5.** Composition de catalyseur selon l'une quelconque des revendications 1 à 4, ladite composition se trouvant dans la plage allant de 10 à 10 000 ppm (M/M) d'un péroxyacide et/ou d'un sel de celui-ci, sur la base du poids total de la composition.

**6.** Composition de catalyseur selon l'une quelconque des revendications 1 à 5, dans laquelle le péroxyacide est choisi parmi l'acide percarboxylique, l'acide perhalique, l'acide hypohalogéneux, l'acide percarbonique, l'acide perborique, l'acide perphosphorique et l'acide persulfurique.

**7.** Composition de catalyseur selon l'une quelconque des revendications 1 à 6, dans laquelle le sel de péroxyacide est choisi parmi les sels d'ammonium, de métal alcalin et de métal alcalino-terreux.

**8.** Procédé d'alcoxylation pour la préparation d'alcoxylates de gamme étroite, ledit procédé consistant à mettre en contact et faire réagir un ou plusieurs réactifs d'oxyde d'alkylène avec un ou plusieurs esters d'acide carboxylique, de préférence, des esters d'acide gras ayant un nombre d'atomes de carbone dans la plage de 7 à 39, en la présence d'une quantité catalytiquement efficace de la composition de catalyseur selon l'une quelconque des revendications 1 à 7.

**9.** Procédé selon la revendication 8, dans lequel le réactif d'oxyde d'alkylène est de l'oxyde d'éthylène, de l'oxyde de propylène et/ou de l'oxyde de butylène.

**10.** Procédé selon la revendication 8 ou 9, dans lequel les esters d'acide carboxylique sont choisis parmi les acétates de méthyle, d'éthyle, d'isopropyle, d'isobutyle, de *sec*-butyle, de *tert*-butyle, d'amyle, de *sec*-amyle, d'isoamyle, de néopentyle, de *tert*-amyle, de *sec*-décyle tel que 2-décyle, 3-décyle, 4-décyle et 5-décyle, de *sec*-undécyle, de *sec*-dodécyle tel que 2-undécyle, 3-undécyle, 4-undécyle, 5-undécyle, 2-dodécyle, 3-dodécyle, 4-dodécyle, 5-dodécyle et 6-dodécyle, de *sec*-tridécyle, de *sec*-tétradécyle tel que 2-tridécyle, 3-tridécyle, 4-tridécyle, 5-tridécyle, 6-tridécyle, 2-tétradécyle, 3-tétradécyle, 4-tétradécyle, 5-tétradécyle, 6-tétradécyle, 7-tétradécyle, de *sec*-pentadécyle, de *sec*-hexadécyle, de *sec*-heptadécyle, de *sec*-octadécyle, de *sec*-nonadécyle, de *sec*-éicosyle, de *sec*-hénéicosyle, de *sec*-docosyle, de *sec*-tricosyle, de *sec*-tétracosyle, de *sec*-pentacosyle, de *sec*-hexacosyle, de *sec*-heptacosyle, de *sec*-octacosyle, de *sec*-nonacosyle, de *sec*-triacontyle, de *sec*-hentriacontyle et de *sec*-dotriacontyle, et mélanges de ceux-ci.

FIGURE 1

Ethoxylated Methyl Laurate
EO-distribution of Examples 1 and 2

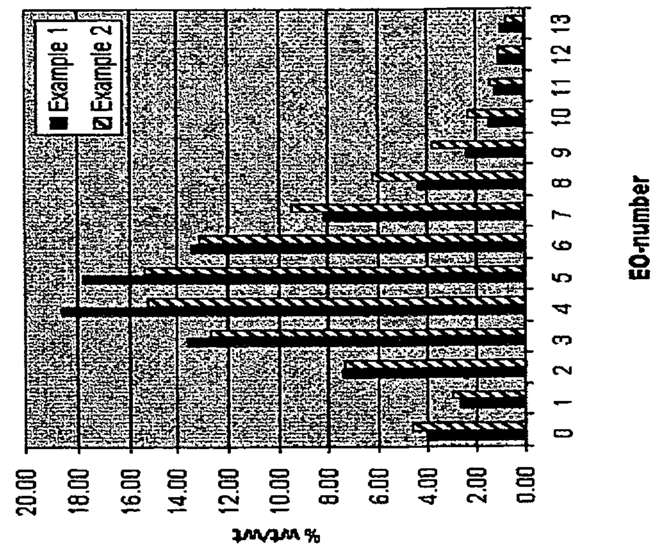

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- GB 1462134 A
- US 4098818 A
- GB 1553561 A
- US 5840995 A
- EP 0335295 A
- WO 0238269 A
- WO 2005115964 A
- WO 02038269 A